Europäisches Patentamt

European Patent Office

Office européen des brevets

(11) Veröffentlichungsnummer: **0 315 085 B1**

# EUROPÄISCHE PATENTSCHRIFT

(45) Veröffentlichungstag der Patentschrift: **30.12.92**

(21) Anmeldenummer: **88118043.4**

(22) Anmeldetag: **29.10.88**

(51) Int. Cl.5: **A61K 39/015**, C12N 15/62, C12N 1/20, C12N 15/30, C12Q 1/68

(54) Rekombinantes histidinreiches Protein von Plasmodium falciparum, seine Herstellung und Verwendung.

(30) Priorität: **03.11.87 DE 3737238**

(43) Veröffentlichungstag der Anmeldung: **10.05.89 Patentblatt 89/19**

(45) Bekanntmachung des Hinweises auf die Patenterteilung: **30.12.92 Patentblatt 92/53**

(84) Benannte Vertragsstaaten: **AT BE CH DE ES FR GB GR IT LI LU NL SE**

(56) Entgegenhaltungen:

**PROCEEDINGS OF THE NATIONAL ACADEMY OF SCIENCES OF THE USA, Band 83, August 1986, Seiten 6065-6069; T.E. WELLEMS et al.: "Homologous genes encode two distinct histidine-rich proteins in a cloned isolate of Plasmodium falciparum"**

(73) Patentinhaber: **BEHRINGWERKE Aktiengesellschaft**
**Postfach 1140**
**W-3550 Marburg 1(DE)**

(72) Erfinder: **Knapp. Bernhard, Dr.**
**Baumgarten 9**
**W-3550 Marburg-Schröck(DE)**
Erfinder: **Hundt, Erika, Dr.**
**Zum Hirtzborn 8**
**W-3550 Marburg-Wehrshausen(DE)**
Erfinder: **Enders, Burkhard, Dr.**
**Oberer Eichweg 12**
**W-3550 Marburg(DE)**
Erfinder: **Küpper, Hans, Dr.**
**Biegenstrasse 39**
**W-3550 Marburg(DE)**

(74) Vertreter: **Fischer, Hans-Jürgen, Dr. et al**
**Hoechst AG Zentrale Patentabteilung Postfach 80 03 20**
**W-6230 Frankurt am Main 80(DE)**

NUCLEIC ACIDS RESEARCH, Band 13, November 1985, Seiten 7837-7846; H.D. STAHL et al.: "Sequence of a cDNA encoding a small polymorphic histidine- and alanine-rich protein from Plasmodium falciparum"

BIOCHEMICAL & BIOPHYSICAL RESEARCH COMMUNICATIONS, Band 146, 15. Juli 1987, Seiten 368-377; R. LENSTRA et al.: "Cloning and sequencing of Plasmodiumfalciparum DNA fragments containing repetitive regions potentially coding for histidine-rich proteins: identification of two overlapping reading frames

The EMBO Journal Band 4 (1985), Seiten 1007-1012.

**Beschreibung**

Auf Grund der zunehmenden Resistenz sowohl des Malariaparasiten Plasmodium falciparum (P. falciparum) gegen altbewährte und neu entwickelte Chemotherapeutika und Prophylaktika als auch seines Überträgers (bestimmte Stechmückenarten) gegen Insektizide wurde Malaria das Hauptgesundheitsproblem in Entwicklungsländern. Die Entwicklung von rekombinanten Proteinen oder synthetischen Peptiden zur Anwendung in der Impfprophylaxe ist deshalb von großer Bedeutung. Ein wichtiger Schritt zur Entwicklung einer modernen Vaccine ist die Identifizierung protektiver Antigene. Als protektiv werden im allgemeinen solche Antigene eingestuft, die bei in vivo Versuchen im Tiermodell wie z.B. in Saimiri- oder Aotus-Affen einen Schutz vor einer intravenös gesetzten P. falciparum-Infektion ergeben haben. Bisher sind nur unbefriedigende Schutzversuche am Menschen beschrieben worden, aber mehrere isolierte P. falciparum-Proteine haben eine komplette oder teilweise Schutzwirkung im Tiermodell gezeigt. Dies trifft sowohl für gelelektrophoretisch gereinigte Proteinbanden von 75 kD und 100 kD zu, als auch für die gelelektrophoretisch gereinigten Proteinfraktionen der Molekulargewichte 200 kD, 140 kD und 41 kD.

Da es nicht möglich sein dürfte, aus Parasitenkulturen die für eine Vaccine benötigten Mengen an Antigenen herzustellen, kommt wohl nur eine gentechnische Herstellung solcher Antigene in Betracht. Zudem kann bei gentechnischer Herstellweise die Unverträglichkeit des Impfstoffs ausgeschlossen werden, die durch Kontamination mit Wirtszellen (erythrozytäre Bestandteile) bedingt ist.

Von den bisher gentechnisch hergestellten Proteinen zeigten ein rekombinantes Expressionsprotein der "5'repeat Region" des sog. RESA 155 kD Merozoitenproteins und ein synthetisches Oligopeptid des 200 kD Merozoitoberflächen-Vorläuferproteins sowie eine Kombination synthetischer Oligopeptide von P. falciparum Proteinen der Molekulargewichte 200 kD, 55 kD und 35 kD in Immunisierungsversuchen mit Saimiri- oder Aotusaffen eine partielle Schutzwirkung.

Die Aufgabe der vorliegenden Erfindung besteht darin, weitere gentechnisch herstellbare P. falciparum-Antigene mit guter Schutzwirkung zu finden, da für eine ausreichende Immunisierung sehr wahrscheinlich eine Mischung mehrerer geeigneter P. falciparum-Antigene erforderlich ist.

Bei dem Versuch, das oben erwähnte protektive 41 kD-Antigen mit Hilfe eines monospezifischen Antiserums gegen diese 41 kD-Proteinbande zu klonieren, wurde aus einer cDNA-Expressionsbank ein Klon 41-3 isoliert, dessen Sequenz in Tab. 1 dargestellt ist. Überraschenderweise codiert diese Sequenz für ein histidin-alanin-reiches Protein von 21 kD, das dem für andere Plasmodienstämme beschriebenen SHARP-bzw. PfHRPIII-Protein vergleichbar ist. (H.D. Stahl et al. (1985), Nucl.Acids Res. 13, 7837-7846; T.E. Wellems und R.J.Howard (1986), Proc. Natl. Acad. Sci U.S.A. 83, 6065-6069). Da es nicht gelang, das Insert des Klons 41-3 befriedigend zur Expression zu bringen, wurde seine Sequenz zum Screening einer weiteren cDNA-Genbank verwendet.

Daraus resultierte durch Kreuz-Hybridisierung ein Klon gt10-41-3, dessen Insert zur Expression gebracht werden konnte.

Die nachfolgend beschriebene DNA-Sequenz dieses Klons (Tab. 2) besitzt einen offenen Leserahmen, der mit dem TAA Stopcodon in Position 568 endet. Eine sehr ausgedehnte "Repeat-Region" (Nukleotide 1 bis 540) codiert fast ausschließlich für Histidin-, Alanin- und Asparaginsäurereste. Die Grundeinheit der "Repeats" bilden Ala-His-His und Ala-Ala-Asp Tripeptid-Repeats.

Die letztere Repeat-Einheit besitzt eine leichte Variabilität: neben 13 Ala-Ala-Asp Tripeptiden findet man drei Ala-Ala-Tyr, zwei Ala-Ser-Asp, zwei Ala-Thr-Asp und eine Val-Ala-Asp Tripeptid-Einheit. Die letzte dieser Einheiten ist ein Dipeptid, dem der Asparaginsäurerest fehlt.

Dagegen sind die Ala-His-His Tripeptid-Repeats äußerst konserviert. Variabilität existiert hier nur in der Anzahl und der Anordnung dieser Tripeptide. Man findet diese zwischen den stets als Monomere vorkommenden Ala-Ala-Asp-Tripeptiden als Monomere (10), Dimere (12) und Trimere (2).

Die hier vorgestellte Sequenz ist der des histidinreichen PfHRPII-Antigens (T.E. Wellems und R.J. Howard (a.a.O.)) sehr ähnlich. Ein Nukleotidsequenzvergleich ergibt einen Homologiegrad von 90 % (Tab. 3). Beide Sequenzen verfügen über ein identisches 3'-Ende von 80 Nukleotiden, differieren aber in der Repeatregion infolge von Deletionen und Basenaustauschen.

Beide Antigene haben einen hohen Anteil an den Aminosäuren Histidin, Alanin und Asparaginsäure und besitzen die Tripeptid-Repeats Ala-His-His und Ala-Ala-Asp. Unterschiede zwischen beiden Sequenzen bestehen hauptsächlich in der Anordnung und der Anzahl der Repeat-Einheiten. So dominiert in der abgeleiteten Aminosäuresequenz des Klons gt10-41-3 das Nonapeptid-Repeat Ala-His- His-Ala-His-His-Ala-Ala-Asp über das Hexapeptid-Repeat Ala-His- His-Ala-Ala-Asp; im PfHRPII Antigen ist dagegen der Hexapeptid-Anteil größer. Generell sind in der hier beschriebenen Sequenz Multimere der Ala-His-His-Tripeptid-Einheiten häufiger gegenüber den im PfHRPII häufigeren entsprechenden Monomeren.

Trotzdem scheint das Insert des Klons λgt10-41-3 für ein HRPII-Antigen zu kodieren, welches in unterschiedlichen P. falciparum-Stämmen heterogen ist. Dies geht unter anderem aus einer Western Blot Analyse mit Antiseren hervor, welche gegen ein Fusionsprotein gerichtet sind, das von dem Vektor pEX 316-41-3 kodiert wird. Diese Seren erkennen mehrere Proteinbanden zwischen 70 kD und 50 kD mit abnehmender Intensität, was sich z. B. durch proteolytische Degradation eines 70 kD-Antigens erklären läßt. Das Molekulargewicht von HRPII liegt - abhängig vom P. falciparum-Stamm - zwischen 70 kD und 75 kD. Gleichzeitig reagiert eine Proteinbande von 37 kD, wobei es sich wahrscheinlich um das homologe HRPIII-Antigen handelt. Ein ähnliches Western Blot Muster wurde von Rock et al. (Rock et al. (1987) Parasitology 95, 209-227) mit Antiseren gegen das HRPII-Antigen gefunden. Die oben beschriebene Nukleotidsequenz des Klons λgt10-41-3 wurde als Fusionsprotein in E. coli zur Expression gebracht.

In einem Vaccinierungsexperiment mit dem gereinigten Expressionsprodukt gelang es, Aotusaffen vor einer P. falciparum-Infektion zu schützen.

Die Erfindung betrifft folglich a) ein Verfahren zur gentechnischen Herstellung des HRPII-Antigens (Stamm SGE2), b) das hierzu erforderliche Gen einschließlich seiner Transkriptionsprodukte, c) dieses Gen ganz oder teilweise enthaltende DNA-Strukturen und Vektoren, d) mit solcher DNA transformierte pro- oder eukaryotische Zellen und das von diesen Zellen exprimierte Polypeptid, e) Aminosäuresequenzen dieses HRPII-Antigens, damit gewonnene Oligopeptide und Antikörper und ihre Anwendung zur Isolierung von Polypeptiden, sowie die Verwendung von HRPII zur Immunisierung und zur Diagnostik von Malaria, f) Impfstoffe gegen Malaria, die HRPII oder immunogene Teile davon alleine oder in Kombination enthalten.

Weitere Ausgestaltungen der Erfindung sind in den nachfolgenden Beispielen und den Patentansprüchen aufgeführt.

Beispiel 1:

Screening einer cDNA-Expressions-Bank mit einem monospezifischen 41 kD Serum

Eine cDNA-Expressionsbank des P. falciparum-Stammes SGE2 (A. Cheung et al. (1985), The EMBO Journal 4, 1007-1012) wurde mit einem polyklonalen Antiserum gegen eine gelelektrophoretisch gereinigte 41 kD Proteinbande (L.H. Perrin et al. (1985), J.Clin.Invest. 75, 1718-1721) nach der Methode von A. Cheung et al. (a.a.O.) gescreent. Daraus resultierten 3 Klone 41-1, 41-2 und 41-3, von denen der Klon 41-3 weiter charakterisiert wurde.

Beispiel 2:

Sequenzierung des Inserts des Klons 41-3

Die DNA-Sequenzierung erfolgte nach der Methode von Maxam und Gilbert (A. Maxam and W. Gilbert (1980), Meth.Enzymol. 65, 499). Die DNA-Sequenz und die daraus abgeleitete Aminosäuresequenz ist in Tab. 1 dargestellt.

Beispiel 3:

Isolierung und Sequenzierung des Klons λgt10-41-3

Da das Insert des Klons 41-3 trotz Insertion in den Vektor pEX31a (K.Strebel et al. (1985), J.Virology 57, 983-991) im richtigen Leserahmen (Überprüfung erfolgte durch Sequenzierung) unter der Kontrolle des hitzeinduzierbaren PL-Promotors als MS2-Polymerase-Fusionsprotein nur schwach exprimiert wurde, wurde mit der Insert-DNA des Klons 41-3 in einer gt10-cDNA Bank nach weiteren Klonen gesucht, um diese zur Expression zu bringen. Die gt10-Bank wurde ausgehend von mRNA des P. falciparum-Stammes SGE2 nach der Methode von T.V.Huynth, R.A. Young und R.W. Davis (in DNA Cloning Vol. I (1985), a practical approach, edited by D.M. Glover, 49-78) unter der Verwendung eines mit den Restriktionsenzymen PstI, NotI und EcoRI spaltbaren 23 bp Linkers hergestellt. Aus dem Screening dieser cDNA-Bank mit der Insert-DNA des Klons 41-3 resultierte der Klon λgt10-41-3. Das Insert dieses Klons wurde nach der Didesoxykettenabbruch-Reaktionsmethode nach F.Sanger et al. (Proc.Natl.Acad.Sci., USA 74, 5463-5467 (1977)) sequenziert. Die Nukleotidsequenz sowie die abgeleitete Aminosäuresequenz dieses Klons ist in Tabelle 2 dargestellt.

Beispiel 4:

4

Expression des Inserts des Klons λgt10-41-3 in dem Vektor pEX31

Das ca. 600 bp umfassende Insertfragment des Klons λgt10-41-3 wurde gelelektrophoretisch isoliert, in den dephosphorylierten, mit dem Restriktionsenzym PstI verdauten Vektor pEX31b ligiert (T. Maniatis et al. (1982) Molecular cloning, A laboratory manual) und nach der Methode von H. Küpper et al. (in Y.Ikeda and T.Beppu (ed). Proceedings of the Fourth International Symposium on Genetics of Industrial Mikroorganisms (1982), Kyoto Kôdansha Ltd., Tokyo), wie in Beispiel 5 im einzelnen ausgeführt, zur Expression gebracht. Das daraus resultierende Plasmid pEX31b-41-3 exprimierte das DNA-Fragment als MS2-Fusionsprotein in hoher Ausbeute. Eine Diskrepanz besteht dabei zwischen dem mittels SDS-Gelen ermittelten Molekulargewicht von 49 kD und dem an Hand der codierenden Sequenzlänge vorhergesagten Molekulargewicht von 33 kD. Möglicherweise ist dieser Effekt durch ein ungewöhnliches SDS-Bindungsverhalten aufgrund der histidinalaninreichen Sequenz bedingt.

Beispiel 5:

Reinigung des Expressionsproduktes

Eine Kultur des das pCl857-Plasmid enthaltende C600-Bakteriums (F.Remaut et al., (1983), Gene 22, 103-113), das mit dem Plasmid pEX31b-41-3 transformiert war, wurde in 1 l LB-Medium mit 50 µg/ml Ampicillin und 25 µg/ml Kanamycin bei 28°C 20 Std. heftig geschüttelt.

Nach Zugabe von 4 l auf 42°C erwärmtem LB-Medium wurde erneut 4 h bei 42°C geschüttelt. Die Bakterien wurden abzentrifugiert, in 200 ml phosphatgepufferter Kochsalzlösung (PBS) resuspendiert und mechanisch aufgeschlossen. Die löslichen Proteine wurden durch Zentrifugation abgetrennt und die Sedimente, die das Expressionsprodukt enthielten, nach zweimaligem Waschen mit PBS in 1 M Harnstoff resuspendiert. Die solubilisierten Proteine wurden durch Zentrifugation abgetrennt und die Sedimente erneut in 2 M Harnstoff suspendiert. Diese Schritte wurden mit steigenden Harnstoffkonzentrationen wiederholt. Bei einer Konzentration von 7 M Harnstoff ging das Fusionsprotein in Lösung und blieb auch bei der Dialyse gegen 2 M Harnstoff löslich, während verunreinigende Komponenten aus E. coli bei diesem Schritt ausfielen. Die abschließend erreichte Reinheit betrug ca. 80%.

Beispiel 6:

Identifizierung des korrespondierenden P. falciparum-Proteins

Kaninchen wurden nach dem folgenden Schema immunisiert: Tag 1: 1 mg gereinigtes Expressionsprodukt in komplettem Freunds Adjuvans, subcutan; Tag 2-5, 15-19, 29-33: je 0,1 mg gereinigtes Expressionsprodukt, intravenös; Tag 40: Blutentnahme.

Mäuse (Balb c), die mit dem Fusionsprotein immunisiert wurden, erhielten am Tag 1 0,03 mg gereinigtes Expressionsprodukt in komplettem Freunds Adjuvans subcutan, am Tag 15 0,1 mg in inkompletten Freunds Adjuvans subcutan, am Tag 29 0,1 mg subcutan und wurden am Tag 36 entblutet. Zur Gewinnung von Schizonten wurde P. falciparum in Humanerythrozyten kultiviert (W. Trager and J.B. Jensen (1976), Science 193, 673-675) und durch Behandlung mit Sorbit synchronisiert (C. Lambros and J.P. Vanderberg (1979), J.Parasitol. 65, 418-420). Eine Anreicherung der Schizonten auf ca. 90% wurde durch Flotation in $^R$Gelafundin (Braun Melsungen) erzielt (analog G. Pasvol et al. (1978), Ann.Trop.Med.Parasitol. 72, 87-88). Die Schizonten wurden abzentrifugiert, gewaschen, in SDS-Probenpuffer gelöst, 5 min. auf 100°C erhitzt, beschallt und aliquo- tiert eingefroren.

Aliquots der Schizontenlösung wurden mit Hilfe der SDS-PAGE aufgetrennt und auf Nitrocellulose transferiert. Die Membran wurde mit 5% Magermilchpulver in PBS abgesättigt und mit geeigneten Verdünnungen der zu untersuchenden Antiseren, danach mit speziesspezifischen biotinylierten Zweitantikörpern inkubiert. Die gebundenen Antikörper wurden mit Hilfe von Avidin/Biotin-Peroxidase und Diaminobenzidin/$H_2O_2$ lokalisiert.

Beispiel 7:

Schutzversuch im Tiermodell: Immunisierung von Aotus azarae boliviensis (Karyotyp VI)

Dieses Experiment wurde durchgeführt, um die Wirksamkeit der HRPII-Partialsequenz (HRPII-P) bezüglich der Induktion von schützender Immunität in P. falciparum-empfänglichen Affen zu prüfen.

1. Versuchsanordnung 9 gesunde Aotusaffen der o.g. Spezies (1000-1500 g Körpergewicht, männliche und weibliche Tiere aus der Zucht der Behringwerke AG) wurden randomisiert und in 3 Gruppen zu je 3 Tieren aufgeteilt.

Alle 3 Tiere der Gruppe A wurden in Abständen von 3 Wochen 3 x mit jeweils 100 μg des pEX 31b-41-3 Expressionsprodukts (gelöst in PBS) subkutan immunisiert. Als Adjuvans diente eine 10%ige Zumischung von 50% Al(OH)$_3$ + 45% Lecithin + 5% Saponin zum Antigen. Die 3 Tiere der Gruppe B wurden nach dem gleichen Schema mit einem analog gewonnenen MS2-Fusionsprotein 31-1 longer repeat delete (31-1 lrd), das eine N-terminale Teilsequenz des 200 kD Vorläuferproteins eines Merozoitoberflächen-Antigens enthält (US-A 879,076), immunisiert.

3 Tiere der Infektions-Kontrollgruppe erhielten ebenfalls nach o.g. Schema jeweils eine Injektion aus PBS + Adjuvans ohne Antigenkomponente.


Tabelle 1


```
           10                30                50
CCGTTTTTGCTTCCGTACTTTTGTTAGATAACAATAACTCCGAATTTAACAATAACTTGT
  ValPheAlaSerValLeuLeuLeuAspAsnAsnAsnSerGluPheAsnAsnAsnLeuP

            70                90               110
TTAGCAAAAATGCAAAAGGACTTAATTCAAATAAGAGATTATTACACGAAAGTCAAGCAC
heSerLysAsnAlaLysGlyLeuAsnSerAsnLysArgLeuLeuHisGluSerGlnAlaH

           130               150               170
ATGCAGGTGATGCCCATCATGCACATCATGTAGCTGATGCTCATCATGCTCACCATGCAG
isAlaGlyAspAlaHisHisAlaHisHisValAlaAspAlaHisHisAlaHisHisAlaA

           190               210               230
CTAATGCTCACCATGCAGCTAATGCTCACCATGCAGCTAATGCTCATCATGCAGCTAATG
laAsnAlaHisHisAlaAlaAsnAlaHisHisAlaAlaAsnAlaHisHisAlaAlaAsnA

           250               270               290
CTCACCATGCAGCTAATGCTCATCATGCAGCTAATGCTCGCCATGCAGCTAATGCTCACC
laHisHisAlaAlaAsnAlaHisHisAlaAlaAsnAlaArgHisAlaAlaAsnAlaHisH

           310               330               350
ATGCAGCTAATGCTCACCATGCAGCTGATGCTAATCACGGATTTCATTTTAACCTTCACG
isAlaAlaAsnAlaHisHisAlaAlaAspAlaAsnHisGlyPheHisPheAsnLeuHisA

           370               390               410
ATAACAATTCCCATACTTTACATCATGCAAAAGCTAATGCTTGTTTTGATGATTCTCACC
spAsnAsnSerHisThrLeuHisHisAlaLysAlaAsnAlaCysPheAspAspSerHisH

           430               450               470
ATGACGATTCCCACCATGATGGAGCACACCACGACGATGCCCACCATGATGGAGCACACC
isAspAspSerHisHisAspGlyAlaHisHisAspAspAlaHisHisAspGlyAlaHisH

           490
ACGACGATGCCCACCAT
isAspAspAlaHisHis
```


6

Tabelle 2

```
        10                  30                  50
CATGTAGCCGATGCCCATCATGCTCATCATGCAGCCGATGCCCATCATGCTCATCATGCA
HisValAlaAspAlaHisHisAlaHisHisAlaAlaAspAlaHisHisAlaHisHisAla

        70                  90                  110
GCCGATGCCCATCATGCTCATCATGCAGCCTATGCCCATCATGCTCATCATGCAGCCGAT
AlaAspAlaHisHisAlaHisHisAlaAlaTyrAlaHisHisAlaHisHisAlaAlaAsp

        130                 150                 170
GCCCATCATGCTCATCATGCTCACCATGCAGCCGATGCCCATCACGCTCATCATGCAGCC
AlaHisHisAlaHisHisAlaHisHisAlaAlaAspAlaHisHisAlaHisHisAlaAla

        190                 210                 230
GATGCCCATCATGCTCACCATGCAGCTGATGCTCATCACGCTCATCATGCAGCCGATGCC
AspAlaHisHisAlaHisHisAlaAlaAspAlaHisHisAlaHisHisAlaAlaAspAla

        250                 270                 290
CATCATGCTCATCATGCAGCCGATGCCCATCATGCTCACCATGCATCCGATGCTCATCAT
HisHisAlaHisHisAlaAlaAspAlaHisHisAlaHisHisAlaSerAspAlaHisHis

        310                 330                 350
GCAGCTGATGCTCACCATGCAGCCTATGCCCATCATGCTCATCATGCTCATCATGCATCC
AlaAlaAspAlaHisHisAlaAlaTyrAlaHisHisAlaHisHisAlaHisHisAlaSer

        370                 390                 410
GATGCTCATCATGCAGCTGATGCTCACCATGCAGCTTATGCCCATCACGCTCATCATGCA
AspAlaHisHisAlaAlaAspAlaHisHisAlaAlaTyrAlaHisHisAlaHisHisAla

        430                 450                 470
GCTGATGCTCATCATGCAGCCGATGCCCATCATGCTCACCATGCAACCGATGCTCATCAC
AlaAspAlaHisHisAlaAlaAspAlaHisHisAlaHisHisAlaThrAspAlaHisHis

        490                 510                 530
GCTCACCATGCAGCCGATGCTCACCATGCAACCGATGCTCACCATGCAGCCGCACACCAC
AlaHisHisAlaAlaAspAlaHisHisAlaThrAspAlaHisHisAlaAlaAlaHisHis

        550                 570
GAAGCCGCCACACATTGCCTACGCCATTAAATTTATTTAATAA
GluAlaAlaThrHisCysLeuArgHisEnd
```

Tabelle 3

```
   1 CATGTAGCCGATGCCCATCATGCTCATCATGCAGCCGATGCCCATCATGC  50
     ||||  ||||||||||||||||||||||  ||||||||  |||||  |||||  ||
 354 CATGCAGCCGATGCCCATCATGCTCACCATGCAGCTGATGCTCATCACGC 403

  51 TCATCATGCAGCCGATGCCCATCATGCTCATCATGCAGCCTATGCCCATC 100
     ||||||||||||||||||||||||||||||||||||||||||||||||||
 404 TCATCATGCAGCCGATGCCCATCATGCTCATCATGCAGCCTATGCCCATC 453

 101 ATGCTCATCATGCAGCCGATGCCCATCATGCTCATCATGCTCACCATGCA 150
     |||||||||||||||  |||||||  ||||||||     |  |||||||||||||
 454 ATGCTCATCATGCATCCGATGCTCATCATGCAGCTGATGCTCACCATGCA 503

 151 GCCGATGCCCATCACGCTCATCATGCAGCCGATGCCCATC.........A 191
     ||  ||||||||||||||||||||||||||||  |||||  ||||  |             |
 504 GCTTATGCCCATCACGCTCATCATGCAGCTGATGCTCATCATGCAGCTGA 553

 192 TGCTCACCATGCAGCTGATGCTCATCACGCTCATCATGCAGCCGATGCCC 241
     ||||||||||||||||  ||||  ||||||||||  |||||  |
 554 TGCTCACCATGCAGCTTATGCCCATCACGCTCATCATGCAGCTGATGCTC 603

 242 ATC.........ATGCTCATCATGCAGCCGATGCCCATCATGCTCACCAT 282
     |||            ||||||||  ||||||  ||||||||  |||||  ||||||||||
 604 ATCATGCAGCCGATGCTCACCATGCAACCGATGCTCATCACGCTCACCAT 653

 283 GCATCCGATGCTCATCATGCAGCTGATGCTCACCATGCAGCCTATGCCCA 332
     |||  ||||||||||  ||||||  |  |||||||||  |||||||||  ||||  ||
 654 GCAGCCGATGCTCACCATGCAACCGATGCTCATCATGCAGCCGATGCTCA 703

 333 TCATGCTCATCATGCTCATCATGCATCCGATGCTCATCATGCAGCTGATG 382
     |||||        ||||||||||||||  ||||||||||||||||||||||||  ||||
 704 CCATGCAGCCGATGCTCATCATGCAACCGATGCTCATCATGCAGCCGATG 753

 383 CTCACCATGCAGCTTATGCCCATC.........ACGCTCATCATGCAGCT 423
     ||||||||||  |    ||||  ||||                |  ||||||  ||||||||
 754 CTCACCATGCAACCGATGCTCATCATGCAGCCGATGCTCACCATGCAGCC 803

 424 GATGCTCATCATGCAGCCGATGCCCATCATGCTCACCATGCAACCGATGC 473
     |||||||||  |||||||  |||||  |  ||||||  ||||||||||||  ||||||||
 804 GATGCTCACCATGCAACCGATTCTCATCACGCTCACCATGCAGCCGATGC 853

 474 TCATCA......CGCTCACCATGCAGCCGATGCT..............C 502
     |·|||||          |||  |||||||||  |  ||||||||                      |
 854 TCATCATGCAGCCGCACACCATGCAACTGATGCTCACCATGCAGCCGCAC 903

 503 ACCATGCAACCGATGCTCACCATGCAGCCGCACACCACGAAGCCGCCACA 552
     ||||||||||||||||||||||||||||||||├|||||||||||||||||||||||||||||||
 904 ACCATGCAACCGATGCTCACCATGCAGCCGCACACCACGAAGCCGCCACA 953

 553 CATTGCCTACGCCATTAAATTTATTTAATAA 583
     |||||||||||||||||||||||||||||||
 954 CATTGCCTACGCCATTAAATTTATTTAATAA 984
```

Tabelle 4

| Gruppe | Anzahl Aotus | $\mu$g Antigen 3 x Immunis. Gesamtdosis | Adjuvans | Challenge Infektion 55.T.p.1.Imm. |
|---|---|---|---|---|
| A HRPII-P | 3 | 300 | modifiziertes Al(OH)$_3$ | $5 \times 10^6$ parasit.Ery. P.falciparum Stamm Palo Alto aus splenektom. Spenderaffen |
| B 31-1 lrd | 3 | 300 | | |
| C Infektionskontr. | 3 | 0 | | |

Um eine möglichst gleichstarke experimentelle P.falciparum-Infektion in den Tieren zu gewährleisten, wurden alle Affen sechs Tage nach der letzten Immunisierung splenektomiert (erhöhte Suszeptibilität). Ein Tier der Kontrollgruppe verstarb vor der Malaria-Infektion interkurrent an den Folgen der Splenektomie.

Am 58. Tag nach der 1. Vaccinierung wurden alle Aotus mit $5 \times 10^6$ parasitierten Erythrozyten intravenös infiziert. Als Challenge-Stamm wurde P. falciparum-Palo Alto gewählt, der, in vitro auf Aotus-Erythrozyten adaptiert, von einem splenektomierten Spendertier (16% Parasitämie) direkt auf die Versuchstiere übertragen wurde. Dieser Stamm zeichnet sich durch eine hohe Infektiosität im Vergleich zu anderen P. falciparum-Subspezies aus. Es ist außerdem interessant zu erwähnen, daß dieser Stamm heterolog zu dem für die Antigenisolierung verwendeten Stamm SGE-2 (Genf) ist (Provenienz, Serotyp, Enzymmuster).

Während der gesamten Verlaufsstudie (vor und nach Immunisierung sowie nach Challenge) wurden physiologische, parasitologische, serologische und klinisch-chemische Parameter untersucht.

2. Ergebnisse Während der gesamten Immunisierungsphase zeigten sich keine pathologischen Veränderungen aller untersuchten physiologischen (klinisches Erscheinungsbild, Temperatur, Gewicht) und klinischchemischen Parameter (Erythrozyten, Hämatokrit, Blutsenkung, Serumenzyme GPT und GOT). Zusätzliche sicherheitspharmakologische Untersuchungen (akute subkutane Toxizität in der Maus, lokale Verträg- lichkeit am Affen nach Richtlinien der Europäischen Pharmakopoe) bescheinigten den verwendeten Impfstoff-Präparationen ausreichende Sicherheit und Verträglichkeit.

2.1 Parasitämie

Hauptparameter für die Wertbemessung eines induzierten Schutzes (Protektion) ist der mikroskopische Nachweis parasitierter Erythrozyten im peripheren Blut des Versuchstieres.

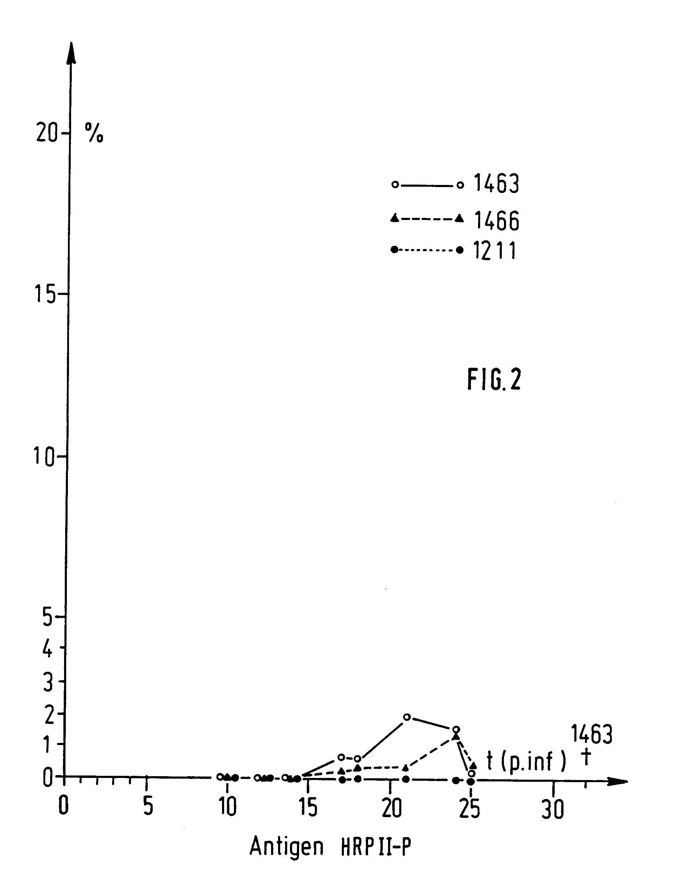

Bereits vom 7.-10. Tag p.i. konnten vereinzelt parasitierte Erythrozyten (weniger als 1 Promille) im Giemsagefärbten Blutausstrich der nicht immunisierten und der mit Antigen 31-1 lrd vaccinierten Tiere nachgewiesen werden. Die mit Antigen HRPII-P immunisierten Tiere zeigten ein verzögertes Auftreten von Parasiten vom 10.-15. Tag p.i. (Fig. 2).

Gemessen an dem Grad der Parasitämie (und auch im Vergleich zu literaturbekannten Daten) sind alle Affen, die mit dem HRPII-P immunisiert wurden, geschützt (keine sterile Immunität). Die verzögert auftretende Parasitämie zeigte bei 2 Tieren maximale Werte von 1-2%, die nach 5 Tagen spontan zu Freisein von Parasiten führte, während Affe Nr. 1211 bis auf eine kurz auftretenden Parasitämie von 1 Promille am Tag 10 p.i. über den gesamten Beobachtungszeitraum parasitologisch negativ blieb (siehe Fig. 2).

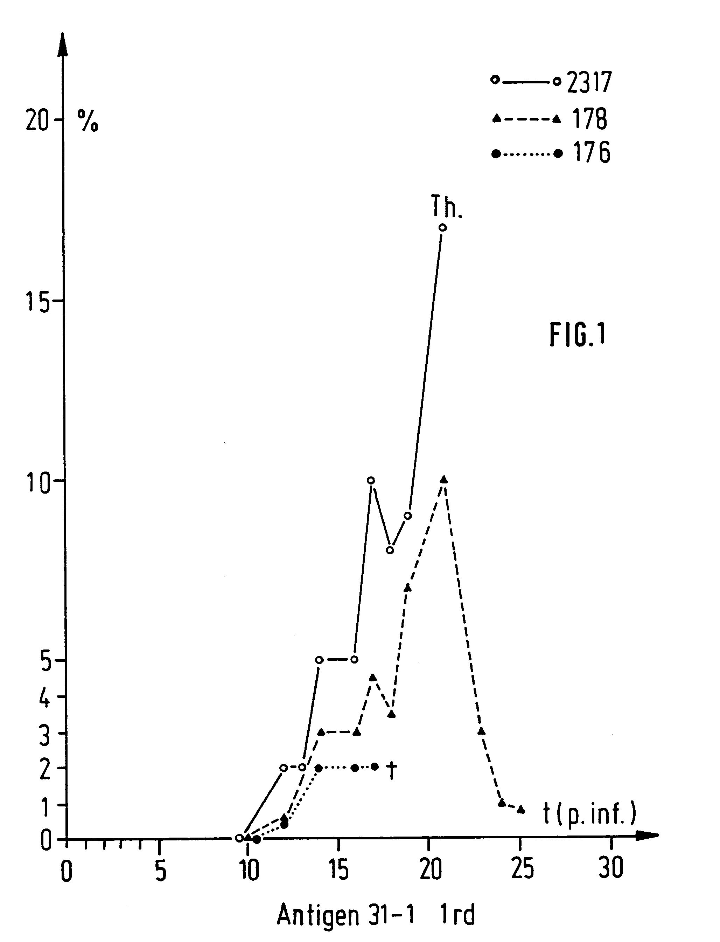

Im Gegensatz dazu waren die mit dem Polypeptid 31-1 lrd immunisierten Tiere ungeschützt (Fig. 1). Während ein Tier eine maximale Parasitämie von 10% selbst kontrollieren konnte, mußte Affe Nr. 2317 einer oralen Chemotherapie mit [R]Mefloquin (Hoffmann La Roche) unterzogen werden, um einem tödlichen Verlauf der Infektion vorzubeugen. Der Challengestamm "Palo Alto" erwies sich in vorangegangenen Infektionsversuchen als [R]Chloroquin-resistent.

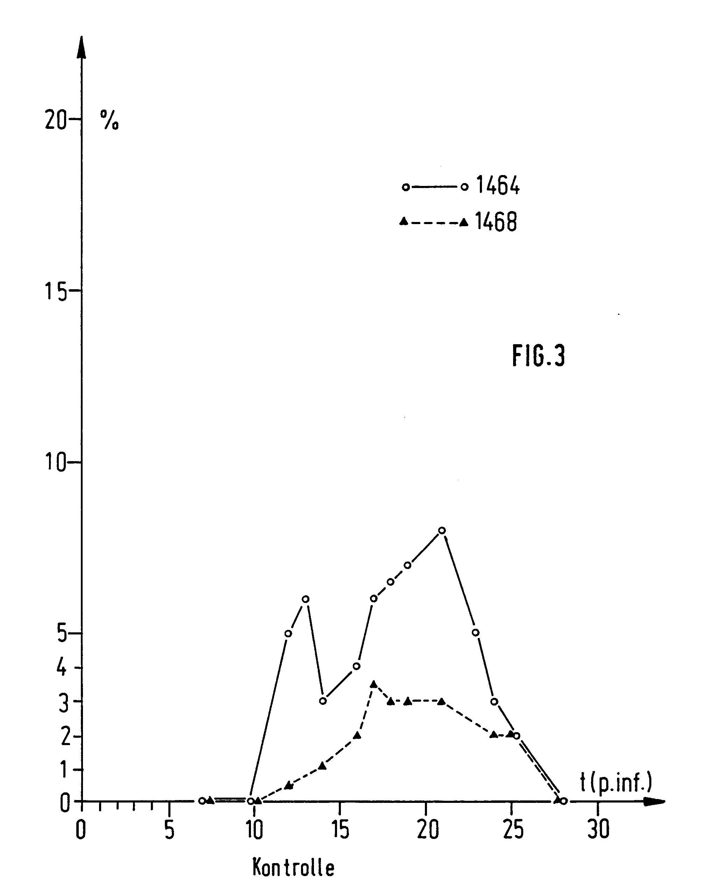

Die nicht immunisierten Affen (Fig. 3) zeigten maximale Parasitämien zwischen 3 und 8%, die, verglichen mit der rasch abklingenden Parasitämie der HRPII-P-immunisierten Tiere, erst innerhalb von 15 Tagen ohne Therapie kontrolliert wurden.

In Fig. 1 bis 3 ist jeweils auf der Ordinate die relative Anzahl der befallenen Erythrozyten (= % Parasitämie) aufgetragen. Die Abszisse stellt die Zeitachse dar (= Tage nach der Infektion).

2.2 Serologie

Antikörper gegen die Immunisierungsantigene wurden mit Hilfe des ELISA bestimmt. 96 well-Polystyrolplatten wurden mit den Fusionsproteinen 31-1 lrd und HRPII-P beschichtet und mit den Seren der immunisierten Affen (1:100 verdünnt) inkubiert; nach Waschen zur Entfernung nicht gebundener Reaktanden wurden spezifische Antikörper mit Peroxidase-markiertem anti-human-IgG vom Kaninchen nachgewiesen.

Die malaria-spezifische Immunantwort wurde ferner mit Hilfe der indirekten Immunfluoreszenz kontrolliert und bestätigt. Aceton-fixierte P. falciparum-infizierte Humanerythrozyten wurden mit Serienverdünnungen der zu untersuchenden Seren inkubiert, die Bindung spezifischer Antikörper wurde mit FITC-markiertem anti-human-Ig vom Kaninchen sichtbar gemacht.

Alle immunisierten Tiere zeigten eine Serumkonversion durch Bildung spezifischer humoraler Antikörper, die mit beiden serologischen Methoden nachgewiesen wurde. Lediglich die gegen das HRPII-PAntigen gerichteten Antikörper zeigten eine Korrelation mit Protektion, während sich das 31-1 lrd-Polypeptid nur als starkes Immunogen ohne induzierende Schutzwirkung erwies. Die Ergebnisse der parasitologischen und serologischen Untersuchungen sind in Tab. 5 zusammengefasst.

Tabelle 5

| Parasitologische und serologische Befunde in Aotus, immunisiert mit rekombinanten Polypeptiden HRPII-P und 31-1 lrd-Antigen aus P.falciparum | | | | | | |
|---|---|---|---|---|---|---|
| Aotus Nr. | Antigen | Max. Parasitämie | | Serologie a.T.d.Infek. | | |
| | | % infiz. Erythr. | Tage nach Challenge | I F T | ELISA 37 kD | ELISA 31-1 lrd |
| 1211 | HRPII-P | 1 % | 10 | 1:80 | 0.6 | n.d. |
| 1466 | | 1.5 | 24 | 1:40 | 0.5 | n.d. |
| 1463 | | 2 | 21 | 1:40 | 0.6 | n.d. |
| 2317 | 31-1 lrd | 17 | 21 | 1: 10 | n.d. | 0.6 |
| 178 | | 10 | 21 | 1: 80 | n.d. | 0.8 |
| 176*) | | 2 | 17 | 1:320 | n.d. | 0.7 |
| 1468 | Infekt. Kontrolle | 4 | 17 | neg. | 0.03 | 0.02 |
| 1464 **) | | 8 | 21 | neg. | 0.03 | 0.03 |

*) am Tag 17 p.i. interkurrent verstorben (Peritonitis)
**) ein Tier starb vor Infekt. an den Folgen der Splenektomie n.d.: nicht durchgeführt

Legende

Tab. 1:    DNA-Sequenz und abgeleitete Aminosäuresequenz des Klons 41-3 von dem P.falciparum Stamm SGE2.

Tab. 2:    Partielle DNA-Sequenz und abgeleitete Aminosäuresequenz der Insert-DNA von gt10-41-3.

Tab. 3:    Nukleotidsequenzvergleich des HRPII-Antigens des P.falciparum Stammes SGE2 (oben) mit dem HRPII Antigen des P.falciparum des Klons 7G8 IMTM 22 (unten).

Fig. 1 bis 3:    Parasitämieverlauf in Aotusaffen, die mit den rekombinanten Proteinen 31-1 lrd (1) bzw. HRPII-P(2) immunisiert wurden. 3: Kontrollen. Auf der Ordinate ist der Anteil der infizierten Erythrozyten (%) aufgetragen.

**Patentansprüche**
**Patentansprüche für folgende Vertragsstaaten : AT, BE, CH, DE, FR, GB, IT, LI, LU, NL, SE**

1.    HRPII-Antigen des Plasmodium falciparum-Stammes SGE2 nach Tabelle 2 und antigen wirksame Teilsequenzen davon, die nicht mit antigen wirksamen Teilsequenzen des Plasmodium faciparum-Stammes IMTM 22 Klon 7G8 identisch sind.

2.    HRPII-Antigen nach Anspruch 1, gekennzeichnet durch Expression von λgt 10-41-3.

3.    Fusionsproteine, die Antigene nach Anspruch 1 enthalten.

EP 0 315 085 B1

**4.** DNA und RNA, kodierend für Antigene nach Anspruch 1.

**5.** Vektoren und DNA-Strukturen, die für Antigene nach Anspruch 1 oder Fusionsproteine nach Anspruch 3 kodieren.

**6.** Wirtszellen, enthaltend DNA nach Anspruch 4 oder einen Vektor nach Anspruch 5.

**7.** HRPII-Antigen, exprimiert von einer Wirtszelle nach Anspruch 6.

**8.** Verwendung von HRPII-Antigen oder immunogenen Teilen davon aus P. falciparum zur Herstellung von Vaccinen.

**9.** Vaccine, enthaltend HRPII-Antigen aus P. falciparum oder immunogene Teile davon.

**10.** Vaccine, enthaltend eines oder mehrere der Antigene nach Anspruch 1, 2 oder 3.

**11.** Diagnostika, enthaltend DNA oder RNA nach Anspruch 4 oder dazu komplementäre DNA oder RNA.

**12.** Diagnostika, enthaltend Antigene nach Anspruch 1, 2 oder 3.

**Patentansprüche für folgende Vertragsstaaten : ES, GR**

**1.** Verfahren zur Herstellung von HRPII-Antigen von Plasmodium falciparum SGE2, dadurch gekennzeichnet, daß die Sequenz der Tabelle 2 oder antigen wirksame Teilsequenzen davon, die nicht mit antigen wirksamen Teilsequenzen des Plasmodium falciparum-Stammes IMTM 22 Klon 7G8 identisch sind, in geeigneten Expressionssystemen exprimiert wird.

**2.** Verfahren zur Herstellung einer Vaccine, dadurch gekennzeichnet, daß HRPII-Antigen aus P. falciparum oder antigen wirksame Teilsequenzen davon eingesetzt wird.

**3.** Verfahren zur Herstellung eines Diagnostikums, dadurch gekennzeichnet, daß die Nukleotidsequenz von Tabelle 2 oder die dazu komplementäre, oder Antigene nach Anspruch 1 eingesetzt werden.

**Claims**

**Claims for the following Contracting States : AT, BE, CH, DE, FR, GB, IT, LI, LU, NL, SE**

**1.** An HRPII antigen of the Plasmodium falciparum strain SGE2 as shown in Table 2 and partial sequences thereof having antigenic activity, which are not identical with partial sequences having antigenic activity of the Plasmodium falciparum strain IMTM 22 clone 7G8.

**2.** The HRPII antigen as claimed in claim 1, obtained by expression of λgt 10-41-3.

**3.** A fusion protein which contains antigens as claimed in claim 1.

**4.** A DNA or RNA coding for antigens as claimed in claim 1.

**5.** A vector or DNA structure which codes for antigens as claimed in claim 1 or fusion proteins as claimed in claim 3.

**6.** A host cell containing DNA as claimed in claim 4 or a vector as claimed in claim 5.

**7.** An HRPII antigen expressed by a host cell as claimed in claim 6.

**8.** The use of HRPII antigen or immunogenic parts thereof from P. falciparum for the preparation of vaccines.

**9.** A vaccine containing HRPII antigen from P.falciparum or immunogenic parts thereof.

11

**10.** A vaccine containing one or more of the antigens as claimed in claim 1, 2 or 3.

**11.** A diagnostic aid containing DNA or RNA as claimed in claim 4, or DNA or RNA complementary thereto.

**12.** A diagnostic aid containing antigens as claimed in claim 1, 2 or 3.

**Claims for the following Contracting States : ES, GR**

**1.** A process for the preparation of HRPII antigen from Plasmodium falciparum SGE2, which comprises expressing the sequence of Table 2 or partial sequences thereof having antigenic activity, which sequences are not identical to partial sequences having antigenic activity of Plasmodium falciparum strain IMTM 22 clone 7G8, in suitable expression systems.

**2.** A process for the production of a vaccine, which comprises using HRPII antigen from P. falciparum or partial sequences thereof having antigenic activity.

**3.** A process for the production of a diagnostic aid, which comprises using the nucleotide sequence of Table 2 or that complementary to this, or antigens as claimed in claim 1.

**Revendications**
**Revendications pour les Etats contractants suivants : AT, BE, CH, DE, FR, GB, IT, LI, LU, NL, SE**

**1.** Antigène HRPII de la souche SGE2 de Plasmodium falciparum, selon le tableau 2, et séquences partielles de celui-ci, exerçant une activité d'antigène, qui ne sont pas identiques aux séquences partielles, exerçant une activité d'antigène, de la souche IMTM 22, clone 7G8, de Plasmodium falciparum.

**2.** Antigène HRPII selon la revendication 1, caractérisé par l'expression de λgt 10-41-3.

**3.** Protéines de fusion qui contiennent des antigènes selon la revendication 1.

**4.** ADN et ARN codant pour des antigènes selon la revendication 1.

**5.** Vecteurs et structures d'ADN, qui codent pour des antigènes selon la revendication 1, ou pour des protéines de fusion selon la revendication 3.

**6.** Cellules-hôtes contenant de l'ADN selon la revendication 4 ou un vecteur selon la revendication 5.

**7.** Antigène HRPII, exprimé par une cellule-hôte selon la revendication 6.

**8.** Utilisation d'antigène HRPII, ou de fractions immunogènes de celui-ci, dérivé de P. falciparum, pour fabriquer des vaccins.

**9.** Vaccins contenant l'antigène HRPII dérivé de P. falciparum ou des fractions immunogènes de celui-ci.

**10.** Vaccins contenant un ou plusieurs antigènes selon la revendication 1, 2 ou 3.

**11.** Agents de diagnostic contenant de l'ADN ou de l'ARN selon la revendication 4, ou l'ADN ou l'ARN qui leur sont complémentaires.

**12.** Agents de diagnostic contenant des antigènes selon la revendication 1, 2 ou 3.

**Revendications pour les Etats contractants suivants : ES, GR**

**1.** Procédé pour préparer l'antigène HRPII de Plasmodium falciparum SGE2, caractérisé en ce qu'on exprime la séquence du tableau 2, ou des séquences partielles de celle-ci, exerçant une activité d'antigène, qui ne sont pas identiques aux séquences partielles, exerçant une activité d'antigène, de la souche IMTM 22, clone 7G8, de Plasmodium falciparum, dans des systèmes d'expression appropriés.

**2.** Procédé pour préparer un vaccin, caractérisé en ce qu'on utilise l'antigène HRPII dérivé de P. falciparum ou des séquences partielles de celui-ci, exerçant une activité d'antigène.

**3.** Procédé pour préparer un agent de diagnostic, caractérisé en ce qu'on utilise la séquence de nucléotides du tableau 2 ou celle qui lui est complémentaire, ou des antigènes selon la revendication 1.

FIG.1

Antigen 31-1   1rd

FIG.2

FIG.3